# EUROPEAN PATENT SPECIFICATION

(11) **EP 2 425 844 B1**
(45) Date of publication and mention of the grant of the patent: **07.05.2014**
(21) Application number: 09844076.1
(22) Date of filing: 09.06.2009
(51) Int. Cl.: A61K 35/28, C12N 5/00

(54) **AUTOLOGOUS AND ALLOGENIC ADIPOSE-DERIVED STROMAL STEM CELL COMPOSITION FOR TREATING FISTULAS**
AUTOLOGE UND ALLOGENE STROMA-STAMMZELLEN-ZUSAMMENSETZUNG AUS FETTGEWEBE ZUR BEHANDLUNG VON FISTELN
COMPOSITION DE CELLULES SOUCHES STROMALES ISSUES DE TISSUS ADIPEUX AUTOLOGUES ET ALLOGÈNES, UTILISABLES POUR LE TRAITEMENT DE FISTULES

(30) Priority: 28.04.2009 KR 20090036942
(43) Date of publication of application: 07.03.2012
(73) Proprietor: Anterogen Co., Ltd., Geumcheon-gu, Seoul 153-802 (KR)
(72) Inventor: LEE, Sung-Koo, Seoul 153-023 (KR); KIM, Mi-Hyung, Seoul 153-023 (KR)
(74) Representative: Schrell, Andreas
(86) International application number: PCT/KR2009/003078
(87) International publication number: WO 2010/126194

(56) References cited:
- US-A1- 2006 045 872
- US-A1- 2006 045 872
- GARCIA-OLMO, D. ET AL.: 'Treatment of enterocutaneous fistula in Crohn's disease with adipose-derived stem cells: a comparision of protocols with and without cell expansion' INT. J. COLORECTAL DIS. vol. 24, 2009, pages 27 - 30, XP019656732
- SANZ-RUIZ, R. ET AL.: 'Adipose tissue-derived stem cells: the friendly side of a classic cardiovascular foe' J. OF CARDIOVASC. TRANS. RES. vol. 1, 2008, pages 55 - 63, XP055098379
- SCHAFFLER, A. ET AL.: 'Concise review: adipose tissue-derived stromal cells- basic and clinical implications for novel cell-based therapies' STEM CELLS vol. 25, 2007, pages 818 - 827, XP009133278

## Description

### [Technical Field]

The present invention relates to a method for production of human adipose tissue-derived stromal stem cells in clinically effective quantities in order to treat fistulas, as well as a composition produced by the same, wherein the stromal stem cells may be obtained from autologous and allogenic adipose tissues.

### [Background Art]

Regenerative medicine refers to a method for treatment of tissues and/or organs with damage or deteriorated function, and is generally related to a cell-based therapy using multipotent stem cells. Bone marrow-derived stem cells, one of the major kinds of adult stem cells which are used in regenerative medicine, can be proliferated in vitro and differentiated into a variety of cells including, for example, myocytes, cardiocytes, osteocytes, chondrocytes, adipocytes, nerve cells, etc. Additionally, bone marrow-derived stem cells have immunomodulatory activity, thus being applicable to allogenic bone marrow transplantation or usable as immunosuppressive agents for auto-immune diseases.

Since adipose tissue also contains large amount of stem cells, extensive studies on use of adipose stem cells as a transplantation material have recently been implemented. Adipose tissue has considerably large quantities of stem cells as compared to other tissue types (i.e., about 1,000 times more numerous than stem cells isolated from the same amount of bone marrow as that of adipose tissue) and the adipose-derived stem cells are multipotent like bone marrow-derived stem cells to differentiate into chondrocytes, osteocytes, adipocytes, myocytes and so on. In addition, adipose-derived stromal stem cells show similar expression of cell surface marker to bone marrow-derived stem cells and have *in vivo* and *in vitro* immunomodulatory activities to autologous or allogenic immune response.

Fistulas are channels abnormally generated in the body and may be generated in various sites of the body by different causes. Most frequently occurring fistulas are in general present in the intestinal system including the anus and rectum. Anal fistulas are commonly caused by an inflammatory disorder wherein inflammation occurs at the anal gland, spreads to the outside of the skin and exudes discharge such as pus, and accounts for about 20% of anal hemorrhoid.

The anal fistula substantially refers to an abnormal channel between the rectal canal and a perianal skin, and induces abnormal fecal discharge through an open site rather than the anus. This is mostly due to occurrence of anorectal abscesses, which in turn, form fistulas discharging pus. Such formed fistula is referred to as 'fistula-in-ano.' That is, the fistula-in-ano is an elongated hole formed from the rectum or the inside of the anal canal to a perianal skin when the anorectal abscess bursts. The fistula-in-ano may be classified into the following four types in relation to the external anal sphincter having an important role in anal performance:
(1) an inter-sphincteric type fistula-in-ano that has a fistula tract formed between the internal anal sphincter and the external anal sphincter;
(2) a trans-sphincteric type fistula-in-ano that has a fistula tract formed to penetrate through the external anal sphincter;
(3) a supra-sphincteric type fistula-in-ano that has a fistula tract formed above the external anal sphincter; and
(4) an extra-sphincteric type fistula-in-ano that has a primary fistula formed near the rectum above an anal crypt rather than the anal crypt itself.

It is generally known that the major cause of the fistula-in-ano is infection of perianal glands and, in addition, the fistula-in-ano sometimes occurs due to tuberculosis, Crohn's disease, cancer, leukemia, leucopenia, or the like. Crohn's disease is characterized by chronic and recurrent inflammation of the bowels and is designated as a rare disease. Fistulas caused by Crohn's disease may include, for example, recto-vaginal fistulas connected to the vagina, enteric fistulas formed near the intestines, etc., as well as fistula-in-ano.

Most fistulas are remedied through surgical operation. The important object of treating the fistulas in the perianal rectum is to completely treat the disease while retaining normal function of the anal sphincters. However, fistulas often recur even after surgical operation and may cause, for example, fecal incontinence due to disorders of anal function, etc. Therefore, current surgical methods do not provide satisfactory treatment effects.

Fistula operations may include, for example, fistulotomy, seton, use of advanced flap, a muscle filling procedure, use of fibrin glue, or the like. Among these, the fibrin glue has been used as a surgical sealant in general surgery by mixing fibrinogen and thrombin to form a fibrin clot. For the fistula-in-ano, the fibrin glue is injected through an external opening into a fistula tract, so as to fill the fistula tract as well as an internal opening of the fistula-in-ano.

In recent years, as extensive studies in relation to stem cells are conducted, a method for treatment of fistula-in-ano by transplanting stem cells to a fistula site has been conducted. For instance, Mizuno et al. (Plast. Reconstr. Surg. 2002, 109:199-209) demonstrated that adult stem cells isolated from adipose tissue may be differentiated into muscle cells, while Damian et al. (Dis Colon Rectum 2005, 48:1416-1423) demonstrated that adipose stem cells may be safely used to treat fistulas-in-ano of patients suffering from Crohn's disease in Phase I clinical trials. In addition, it was reported that, when patients with Crohn's disease are subjected to transplantation of adult stem cells to treat a rectal fistula, fecal incontinence does not occur even 3 months after transplantation.

In vitro culture of adipose-derived stromal stem cells is known to be easy to implement. However, in order to obtain clinically effective number of cells, a relatively large amount of adipose tissue and a long-term culture period are generally required. Specifically, treatment of fistulas in patients suffering from chronic and/or recurrent diseases such as Crohn's disease requires a massive amount of cells, as well as an improved cell culturing procedure. In addition, if it is allowed to use allogenic adipose-derived stromal cells, they may be clinically very useful.

WO 2006/136244 (December 28, 2006) discloses fistula treatment using adipose-derived stromal stem cells and use thereof. However, since the adipose stem cells used in the treatment are obtained from a patient himself, if the patient does not have enough adipose tissue, the number of stem cells contained in the adipose tissue is considerably small, or it fails to culture autologous stem cells, clinically suitable treatment may not be provided. Especially, in the case of patients with Crohn's fistula, significant enteric inflammation is encountered, inducing weight loss and low body mass index of the patient. For such a patient, it is difficult to ensure a sufficient quantity of adipose-derived stromal stem cells. Moreover, since the disease is continued for a long term and becomes worse to usually cause occurrence of several fistulas in a person and a size of the fistula is relatively increased, a massive amount of stem cells is required to treat the patient.

Meanwhile, for in vitro culture of adipose-derived stromal cells, when standard cell culture methods disclosed in existing patents (WO 2007/011797, US 6,777,231, etc.) or published documents (Tissue Engineering 7(2), 211-228, 2001, etc.) are employed, in vitro cell culture needs a long time, causing difficulty in obtaining clinically effective number of cells. Accordingly, actual efficiency in clinical applications may be deteriorated. Although exact grounds are still unknown, a rate for proliferation of adipose stem cells varies to different individuals and cell culture may be failed since adipose stem cells are not actively proliferated during in-vitro culture. Furthermore, biological functions of adipose stem cells including, for example, multipotency, immunomodulatory activity, or the like, may vary depending upon donors thereof. Therefore, using allogenic adipose stem cells having clinically effective features may improve fistula treatment efficacy.

### [Technical Problem]

Accordingly, the present invention relates to the use of stromal stem cells isolated from human adipose tissues in fistula treatment, and the object of the present invention is to provide an improved method for producing clinically effective number of stromal stem cells, as well as an adipose stem cell composition produced by the foregoing method.

### [Technical Solution]

In order to achieve the object described above, the present invention provides an adipose stem cell composition for treating fistula, comprising allogenic adipose tissue-derived stromal stem cells.

In other to achieve another object described above, the present invention provides a method for production of an adipose stem cell composition comprising of adipose-derived stromal stem cells, the method comprising the steps:
(a) isolating a stromal vascular fraction ('SVF') from adipose tissue collected from a subject;
(b) incubating the SVF in a stromal medium;
(c) incubating the SVF in an expansion medium containing EGF or bFGF as a growth factor, to culture the adipose-derived stromal stem cells; and
(d) culturing the adipose-derived stromal stem cells for at least one passage.

According to the present invention, there are further provided an adipose stem cell composition produced by the foregoing method, which can be used for treating a fistula comprising the step of administering the foregoing composition to a fistula of a patient.

The present invention substantially relates to a method for production of clinically effective quantities of human adipose tissue-derived stromal stem cells in order to treat fistula, and a composition produced by the same. Adipose-derived stromal cells used in transplantation may be isolated from autologous and/or allogenic adipose tissue. More particularly, allogenic adipose-derived stromal cells according to the present invention may be employed in the treatment without isolating adipose tissue from a subject patient and allogenic adipose stem cells having more excellent treatment efficacy may be selected and used in treatment, thereby having clinical suitability.

In addition, with regard to culture of a stromal vascular fraction (hereinafter, often referred to as 'SVF') isolated from autologous or allogenic adipose tissue, the present invention may appropriately use a stromal medium and an expansion medium to implement the culture, thereby effectively producing clinically effective number of stromal stem cells in a short time. More particularly, the present invention may utilize an expansion medium including specific growth factors such as a basic fibroblast growth factor (hereinafter, referred to as 'bFGF') or an epidermal growth factor (hereinafter, referred to as 'EGF'), to thereby noticeably reduce culturing time of stem cells and easily control a growth rate thereof, which varies to different individuals. Moreover, adipose stem cells produced by the inventive method show superior differentiation rate and immunomodulatory activity, compared to ones produced by existing cell culture methods.

If an abundant adipose tissue isolated from a patient by liposuction is provided, adipose derived stem cells may be purified and used. However, in most cases, in order to obtain sufficient amount of adipose-derived stem cells for transplantation, a proliferation stage is needed. That is, after calculating an amount of the adipose tissue obtained by liposuction, the number of adipose tissue-derived stromal stem cells isolated from the adipose tissue and the number of stem cells required for transplantation, cell culture and subculture should be conducted to obtain clinically effective number of stem cells. For allogenic transplantation, a subculture is advisable to sufficiently remove a series of cells which may be induce an immune response such as immune cells, fibroblasts, or the like, and to produce a homogenous population of the stromal stem cells. Preferably, at least one subculture should be performed.

In the present text, "stromal medium" means a medium for incubating the SVF and may be prepared by adding 10% FBS and 1% antibiotics to a proper cell culture medium such as DMEM or DMEM/F12. On the other hand, "expansion medium" refers to a medium used for proliferation of adipose-derived stem cells and may additionally include bFGF or EGF in the stromal medium.

According to a culturing method of the present invention, an average doubling time of adipose-derived stem cells is about 48 hours to thereby attain remarkably enhanced effects, compared to a conventional culturing method (WO 2007/011797) requiring an average doubling time of about 72 to 120 hours. As shown in FIG. 1, average culture period in each passage is 4 days under predetermined conditions according to the present invention while average period for each passage according to the conventional method is 7 days and, therefore, the present invention may considerably decrease days for cell production, that is, the time required to produce a sufficient number of cells. For instance, in order to obtain 1×10⁸ adipose stem cells using 50 ml of donated lipoaspirate, existing culturing methods require at least 30 days. In contrast, when the donated lipoaspirate is incubated in an expansion medium of the present invention that includes bFGF or EGF in a typical stromal medium, a desired product may be obtained in about 15 days. According to another example, if an amount of the donated lipoaspirate is less than 50 ml, the existing culturing method entails difficulties in producing clinically effective number of adipose stem cells, whereas the present invention provides an improved culturing method to overcome such difficulties.

For the culture of stromal stem cells, doubling time considerably varies depending on donors of adipose tissue, however, the present invention may minimize the differences in culturing time due to individual differences between donors. Consequently, a cell culturing method of the present invention may be suitable to ensure clinically effective number of cells for fistula treatment.

In general, it is known that, as the number of generations increases through in vitro culture, multipotency of the stem cells is decreased. Accordingly, a culturing method capable of maintaining desired multipotency of stem cells is required. When the inventive culturing method is used, the stem cells show superior muitopotency to differentiate into myocytes, osteocytes, or the like, compared to existing culturing methods. Also, adipose-derived stromal stem cells obtained according to the inventive culturing method favorably retain inherent immunomodulatory activity and exhibit even more excellent effects. That is, if stem cells are incubated in an expansion medium containing bFGF or EGF according to the present invention, multipotency and immunomodulatory activity as inherent characteristics of the stem cells are better maintained, thus attaining clinically superior effects.

Adipose-derived stromal stem cells are known not to induce immune response to allogenic cells, but to have immunomodulatory activity to inhibit induced-immune response. The present invention proposes possible usage of allogenic adipose stem cells for fistula treatment. For instance, when adipose stem cells are added under conditions for activation of peripheral blood mononuclear cells from patients with Crohn's disease, immune response may be suppressed (or controlled). In this case, allogenic stem cells substantially have functions equal to or better than autologous stem cells. Therefore, the allogenic adipose stem cells may be effectively used to treat fistulas caused by Crohn's disease or other types of fistulas.

Stromal stem cells used for transplantation in the present invention may be prepared through culture for one to ten passages, and preferably at least 50%, and more preferably at least 80% of such stem cells is positive to CD10, C13, CD29, CD44, CD59, CD71, CD90, CD105 and Oct 4, while being negative to CD34, CD45, CD 104, CD 106 and Stro-1.

Details of the present invention will be more clearly understood from the following detailed description.

In the present context, "stromal vascular fraction (SVF)" refers to cells isolated from adipose tissue and means a remaining group of cells after removing most of mature adipocytes by treating adipose tissue with collagenase and centrifuging process.

"Adipose-derived stromal stem cells" mean mesenchymal stem cells obtained from the SVF and may also be designated as "adipose-derived stem cells (ASC)," "adipose-derived adult stem cells (ADAS)," "adipose stem cells," or the like.

"Fistula" as used herein refers to an abnormal channel generated in the body. Examples of such fistula may include fistula-in-ano, anorectal fistula, rectovaginal fistula, bladder fistula, enteric fistula, fecal fistula, or the like, without being particularly limited thereto.

"Allogenic transplantation" means transplantation of a specific tissue, organ or cells from others or other individual of the same species and, more particularly, transplantation of a specific tissue, organ or cells from another person or other animals in the case of where autologous tissue, organ or cells cannot be used.

The present invention primarily includes collecting an SVF from autologous or allogenic adipose tissue and culturing adipose-derived stromal stem cells from the SVF, and such procedures have already been reported. The present invention proposes a method for production of clinically effective number of cells for fistula treatment by improving a conventional method proposed in US Patent No. 6,777,231. More particularly, US Patent No. 6,777,231 discloses a method including; using collagenase to treat adipose tissue obtained by liposuction and prepare an SVF, incubating the obtained cell group, that is, the SVF in a stromal medium that consists of DMEM or DMEM/F12 (Dulbecco's Modified Eagle Medium/Ham's F-12 Nutrient Broth) containing 10% bovine serum, and culturing the incubated cells for at least one passage when they have grown to cover 80 to 90% (of a culture vessel). On the other hand, the present invention includes; incubating an SVF in a stromal medium for 24 hours, and then, subjecting the incubated cells to an expansion medium which is stromal medium containing bFGF or EGF. According to the present invention, the expansion medium may be prepared by adding 0.1 to 100 ng/ml and, preferably, 1 to 10 ng/ml of bFGF or EGF to a stromal medium, to thereby enhance cell proliferation rate while maintaining multipotency and immunomodulatory activity. Therefore, the present invention provides an improved cell production method.

Adipose-derived stromal stem cells in the present invention may be obtained from human subcutaneous fat tissue by liposuction or surgical excision, without being particularly limited thereto.

Autologous and/or allogenic adipose-derived stromal stem cells used for transplantation in the present invention may be obtained according to the following procedures, provided in that a base medium for cell culture is not particularly limited to the following description.

### (1) SVF separation from adipose tissue obtained by liposuction.

After the adipose tissue is washed with a KRB solution and treated with collagenase, centrifugation is executed. Upper layer of lipid is removed and a bottom layer is added with a physiologically suitable saline solution (i.e., phosphate buffer solution (PBS)) to form a suspension. It is followed by centrifugation to recover a bottom layer comprising an SVF.

### (2) Incubation of SVF in stromal medium.

After suspending the SVF in a stromal medium, the suspension is inoculated into a culture vessel to be a concentration of 10,000 to 40,000 cells/cm² and cultured therein. The stromal medium is a medium that includes DMEM or DMEM/F12 (Dulbecco's Modified Eagle Medium/Ham's F-12 Nutrient Broth) containing 10% fetal bovine serum and is used to culture the SVF for 24 hours.

### (3) Incubation in an expansion medium

After removing the stromal medium, expansion medium is added to proliferate adherent (or agglutinant) cells. The expansion medium may be an DMEM or DMEM/F12 including 10% fetal bovine serum and EGF with a concentration of 0.1 to 100 ng/ml or bFGF with a concentration of 0.1 to 100 ng/ml, which functions to accelerate proliferation of adipose-derived (adherent or agglutinant) stromal stem cells, to thereby considerably increase the number of cells in a short time.

### (4) Subculture

When the cells fill 80 to 90% of the bottom of the culture vessel, the expansion medium is removed and trypsin treatment is conducted to harvest the cells from the culture vessel. For subculture, the cells should be diluted in a ratio of 1:3 to 1:4, then, cultured using an expansion medium in a new culture vessel. By the same procedures as described above, further subculture may be performed.

### (5) Confirmation of immunomodulatory activity

Since allogenic adipose-derived stromal stem cells have different immunomodulatory activities depending on types (individuals) thereof, after observing and identifying immunomodulatory activity of adipose-derived stromal stem cells obtained after at least one generation of subculturing, an allogenic adipose-derived stromal cell having excellent immunosuppressive ability may be selected and used, thereby attaining superior clinical effects over autologous cells.

### [Brief Description of the Drawings]

The above and other objects, features and other advantages of the present invention will be more clearly understood from the following detailed description, taken in conjunction with the accompanying drawings, in which:
FIG. 1 is a graph illustrating CPDL of adipose stem cells incubated in a stromal medium or an expansion medium;
FIG. 2 is a graph illustrating yield of adipose stem cells incubated in a stromal medium or an expansion medium for 20 days;
FIG. 3 is a photograph (×40) showing myocyte differentiation of adipose stem cells incubated in a stromal medium or an expansion medium;
FIG. 4 is a photograph (×40) showing oteocyte differentiation of adipose stem cells incubated in a stromal medium or an expansion medium;
FIG. 5 illustrates immunogenicity of adipose stem cells;
FIG. 6 illustrates immunosuppressive activity of adipose stem cells during MLR;
FIG. 7 illustrates IFN-γ secretion suppressive activity of autologous and allogenic adipose stem cells with respect to PHA-stimulated immune response;
FIG. 8 illustrates TNF-α secretion suppressive activity of autologous and allogenic adipose stem cells with respect to PHA-stimulated immune response;
FIG. 9 illustrates both IFN-γ and TNF-α secretion suppressive activity (n=9) of adipose stem cells cultured in a stromal medium and an expansion medium, with respect to PHA-stimulated immune response;
FIG. 10 is photographs (×40) showing extra-cellular matrix protein secretion activity of adipose stem cells; and
FIG. 11 is photographs showing fistula-in-ano before and after transplantation of adipose stem cells.

### [Best Mode]

Hereinafter, preferred embodiments and examples of the present invention will be described in detail. However, these examples are given for the purpose of illustration and are not intended to limit the invention.

### EXAMPLE 1 - Culture method of human adipose-derived stromal stem cells

Adipose tissue is generally obtained by liposuction, without being particularly limited thereto.

Adipose-derived stromal cells were isolated from lipoaspirates according to the following procedures: the obtained adipose tissue was washed three or four times using an equal volume of KRB solution in order to remove blood from the adipose tissue. Adding an equal volume of collagenase solution to the adipose tissue, reaction was carried out in a water bath at 37°C. The reaction product was placed in a centrifuge tube and centrifuged at 20°C and 1200 rpm for 10 minutes. After removing a fat layer as a supernatant, the remaining collagenase solution as a lower layer was gently separated without being shaken. A stromal medium was added to the collagenase solution to prepare a suspension, followed by centrifugation at 20°C and 1200 rpm for 5 minutes. Here, the settlement part was a stromal vascular fraction, that is, SVF, while the supernatant was discarded.

The SVF was suspended in the stromal medium, inoculated into a culture vessel and cultured for 24 hours in an incubator at 37°C under 5% CO₂. After removing the culture medium and washing the SVF with a phosphate buffered saline (hereinafter, 'PBS'), the SVF was proliferated in a stromal medium, a stromal medium containing bFGF with a concentration of 1 ng/ml, or a stromal medium containing EGF with a concentration of 5 ng/ml. When the adipose-derived stromal cells were grown to cover 80 to 90% of the culture vessel, the cells were subjected to trypsin treatment to isolate and obtain single type cells. The obtained single cells were diluted to a ratio of 1:3 to 1:4, using an expansion medium, followed by subculturing.

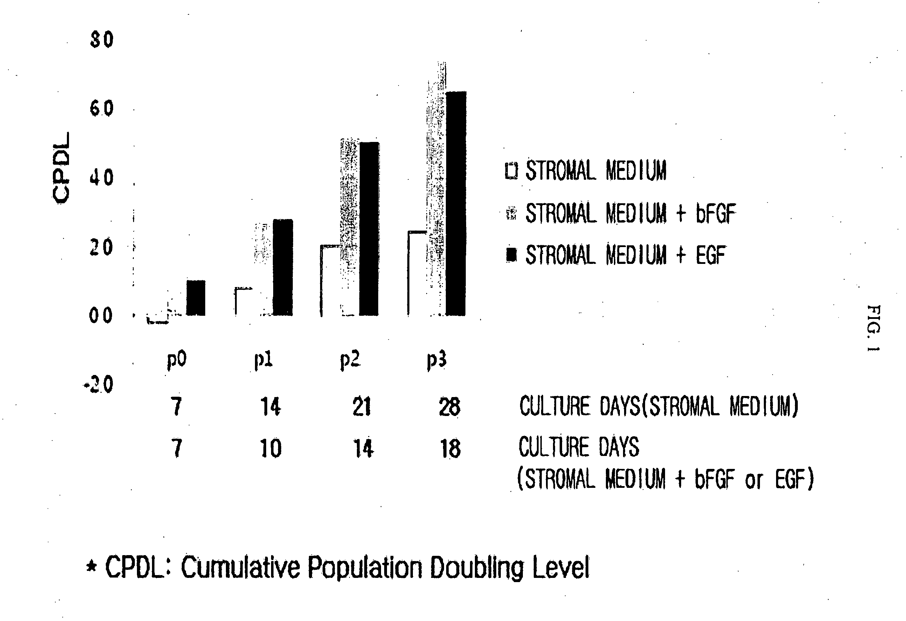

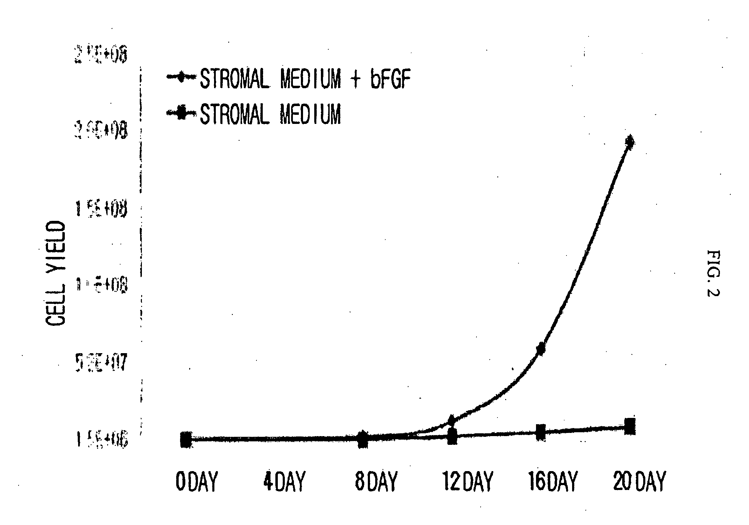

FIG. 1 is a graph illustrating CPDL of adipose stem cells incubated in a stromal medium or an expansion medium, and FIG. 2 is a graph illustrating yield of adipose stem cells incubated in a stromal medium or an expansion medium for 20 days. As shown in these figures, it was found that, if the adipose stem cells are incubated in the expansion medium which is a stromal medium with bFGF or EGF, a growth rate of cells is about 3 times higher than when using a standard medium, that is, the stromal medium alone. When 1.5×10⁶ cells were incubated in each of the foregoing media for 20 days, average cell yield was 9.7× 10⁶ in the stromal medium and 1.9×10⁸ in the expansion medium, respectively. Consequently, it can be seen that the cells in the expansion medium can be collected about 20 times more than in the stromal medium..

The following TABLE 1 shows doubling time and cell yields depending on culture media.

**TABLE 1**

| | Stromal medium | Stromal medium + bFGF |
|---|---|---|
| Doubling time | 122 hours | 48 hours |
| Cell yield* | 9.7×10⁶ | 1.9×10⁸ |

| | | |
|---|---|---|
| (*) An average number of cells obtained by incubating 1.5×10⁶ cells in each medium for 20 days. | | |

It is known that adipose stem cells may undergo subculturing for 10 or more passages, without variation in proliferation rate and phenotype. When a standard culturing method using a stromal medium is used to subculture 1×10⁶ adipose stem cells, the number of cells obtained is about 3.8×10⁹. On the other hand, about 1.5×10¹³ cells are obtained using the expansion medium according to the present invention, which is about 4,000 times the cell number obtained using the foregoing stromal medium.

In the case where 50ml and 100ml of adipose tissues obtained by liposuction are cultured using respective media, culturing times required to produce 1×10⁸ cells are shown in TABLE 2 below.

**TABLE 2**

| | Stromal medium | Stromal medium + bFGF |
|---|---|---|
| 50 ml | Minimum 30 days | Within 15 days |
| 100 ml | Minimum 20 days | Within 13 days |

Although exact causes and/or reasons are not clearly known, a proliferation rate of adipose stem cells varies according to individuals and, since proliferation of adipose-derived stromal cells is not actively implemented during in vitro culture, cell culture may sometimes fail.

The following TABLE 3 shows culturing results of adipose stem cells wherein 50ml of an adipose tissue was obtained from 7 different donors and adipose stem cells were prepared from the adipose tissue and cultured in respective media. When culturing was performed using the expansion medium according to the present invention, a culturing time required to obtain 1×10⁸ cells ranged from 13 to 18 days. On the other hand, when using a stromal medium according to a standard culturing method, culturing time was about 25 to 35 days in four (4) lots. Further, for three (3) lots, cell production was failed because cell proliferation was not sufficient.

**TABLE 3**

| Lot No. | Expansion medium (stromal medium + bFGF) | Stromal medium |
|---|---|---|
| #1 | 16 days | 26 days |
| #2 | 18 days | 35 days |
| #3 | 13.5 days | Cell production was impossible |
| #4 | 13 days | 27.5 days |
| #5 | 13.7 days | 25 days |
| #6 | 14 days | Cell production was impossible |
| #7 | 14.5 days | Cell production was impossible |

### EXAMPLE 2 - Cell surface marker of human adipose-derived stromal stem cells

The dipose-derived stromal cells obtained by the same procedure as described in Example 1 were placed in a 1.5 ml centrifuge tube. After adding 1 ml of a FACS staining solution (a PBS containing 1% fetal bovine serum) thereto and homogeneously mixing the same, centrifugation at 10,000 rpm was conducted for 5 seconds. After removing the supernatant, the remaining product was suspended in 1ml of FACS staining solution and centrifuged at 10,000 rpm for 5 seconds. After removing the supernatant, the remaining product was re-suspended in 300µℓ of the FACS staining solution. The resultant sample was distributed into new centrifuge tubes, such that about 0.5 to 1.0×10⁶ cells are placed in each of the centrifuge tubes, in consideration of the number of samples. After adding an antibody thereto, the content in the tube was subjected to reaction at 4°C for 30 minutes. Then, the reaction product was re-suspended in 1ml of the FACS staining solution and centrifuged at 10,000 rpm for 5 seconds, followed by removal of the supernatant. The remaining product was re-suspended by adding 400 to 500 µℓ of a FACS fixing solution thereto. The obtained suspension was subjected to analysis using a flow cytometer.

As shown in TABLE 4 below, it was found as a result of the analysis that at least 95% of the adipose stem cells proliferated by the present invention exhibited positive response to CD10, CD13, CD29, CD44, CD59, CD71, CD90, CD105 and Oct4, and negative response to STRO-1, CD34, CD45, CD104 and CD 106. However, in case of CD34, about 5 to 10% of the adipose stem cells sometimes exhibited positive response at p0, depending on given adipose tissue lots. Difference based upon culturing conditions has not been observed.

**TABLE 4**

| Passage culture | p0 | | | p1 | | | p2 | | | P3 | | |
|---|---|---|---|---|---|---|---|---|---|---|---|---|
| | Stromal medium | EFG | bFGF | Stromal medium | EFG | bFGF | Stromal medium | EFG | bFGF | Stromal medium | EFG | bFGF |
| CD10 | + | + | + | + | + | + | + | + | + | + | + | + |
| CD13 | + | + | + | + | + | + | + | + | + | + | + | + |
| CD29 | + | + | + | + | + | + | + | + | + | + | + | + |
| CD34 | -/+ | -/+ | -/+ | - | - | - | - | - | - | - | - | - |
| CD44 | + | + | + | + | + | + | + | + | + | + | + | + |
| CD45 | - | - | - | - | - | - | - | - | - | - | - | - |
| CD59 | + | + | + | + | + | + | + | + | + | + | + | + |
| CD71 | + | + | + | + | + | + | + | + | + | + | + | + |
| CD90 | + | + | + | + | + | + | + | + | + | + | + | + |
| CD105 | + | + | + | + | + | + | + | + | + | + | + | + |
| STRO-1 | - | - | - | - | - | - | - | - | - | - | - | - |

### EXAMPLE 3 - Differentiation ability of human adipose-derived stromal stem cells

When the adipose-derived stromal stem cells cultured by the same procedure as described in Example 2 were grown to cover 80 to 90% of a culture vessel, the cultured cells were subjected to trypsin treatment to isolate and obtain single cells.

In order to identify myogenic differentiation ability of the adipose-derived stromal stem cells cultured using each of different media, the cells were inoculated into four (4) wells such that each well contains about 5,000 cells/cm². The inoculated cells were cultured to fill about 80% of the culture vessel while changing the medium. When the adipose-derived stromal stem cells filled 100% of the culture vessel, the used medium was discarded and replaced with myocyte differentiation medium manufactured by Promocell and differentiation of the stem cells into myocytes was induced for 2 weeks. In order to determine myocyte differentiation rate, immuno-fluorescence staining was performed by the following procedures: the stem cells were washed three times using PBS and fixed using PBS containing 4% formaldehyde for 30 minutes. Then, after washing the fixed cells three times with PBS, the washed product was subjected to permeabilization and blocking using PBS containing 5% normal goat serum and 0.1% Triton X-100 for 30 minutes. After adding PBS that includes primary antibodies of Desmin and Myosin which are specific for myocytes to the cells, and then reacting them at 37°C for 1 hour, the reaction product was washed three times with PBS and subjected to reaction using a secondary antibody for 30 minutes. After the product was washed again three times using PBS, a mounting solution was applied thereto and observed by fluorescence microscopy.

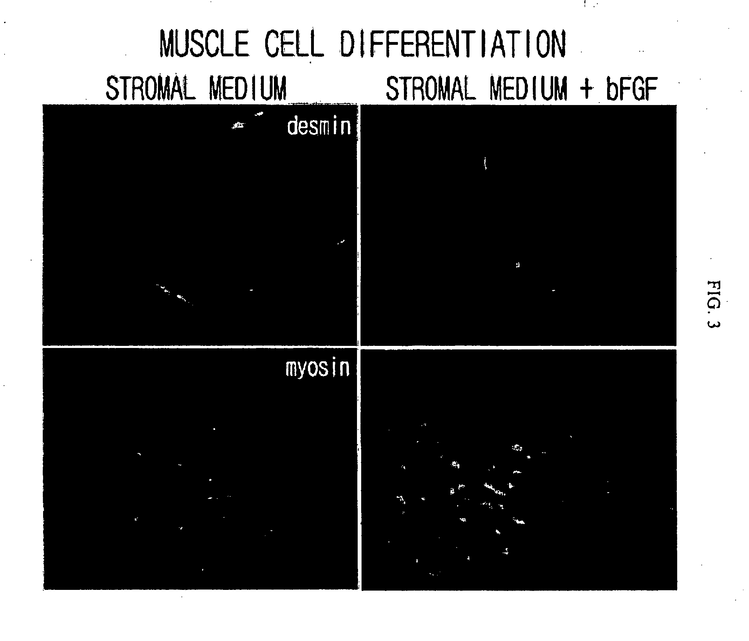

FIG. 3 is photographs (×40) showing myocyte differentiation of adipose stem cells which were cultured in a stromal medium or an expansion medium. As shown in FIG. 3, compared to the culture using the stromal medium, a lot more cells positive to Desmin and Myosin were observed in the adipose-derived stromal stem cells obtained by the culture using the expansion medium containing bFGF. That is, according to the present invention, when the adipose-derived stromal stem cells are incubated in a medium prepared by adding bFGF to the stromal medium, it can be seen that a myocyte differentiation rate is improved.

In order to identify osteocyte differentiation of adipose-derived stromal stem cells cultured using different media, the cells were inoculated into twelve (12) wells such that each well contains about 20,000 cells/cm². The inoculated cells were cultured to fill about 100% of a culture vessel while changing the medium. When the adipose-derived stromal stem cells filled 100% of the culture vessel, the used medium was discarded and replaced with an osteocyte differentiation medium, which includes 100 µg/mℓ of ascorbic acid, 10nM glycerophosphate, 100nM dexamethasone and 10nM vitamin D3, and differentiation of the stem cells into osteocyte was induced for 4 weeks. In order to determine an osteocyte differentiation rate, von-Kossa staining was performed by the following procedures: the stem cells were washed three times using PBS and fixed using PBS containing 4% formaldehyde for 20 minutes. Then, after washing the fixed cells three times using distilled water, they were added with a 5% silver nitrate solution thereto, and was exposed to UV radiation for 1 hour. After washing the exposed material three times using the distilled water and adding a 5% sodium thiosulfate solution thereto, reaction was performed for 2 minutes. After washing the reaction product three times using the distilled water, a mounting solution was applied thereto and the cells were observed using a microscope.

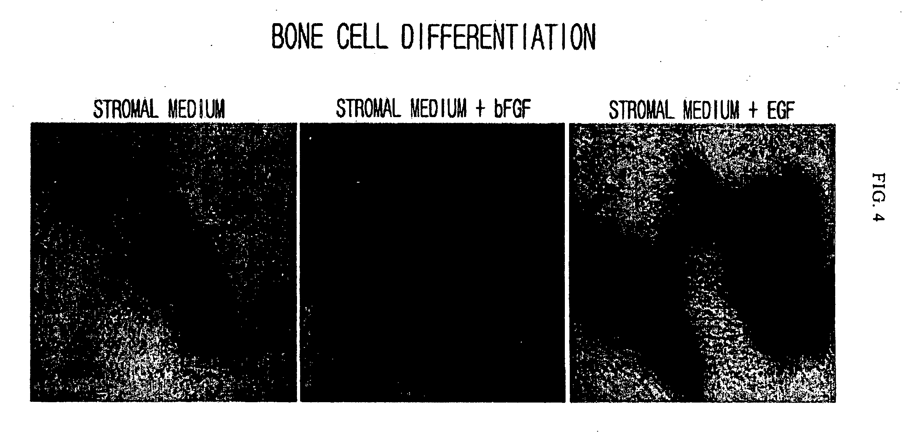

FIG. 4 is photographs (×40) showing osteocyte differentiation of adipose stem cells cultured in a stromal medium or an expansion medium. As shown in FIG. 4, the adipose-derived stromal stem cells obtained by culturing using the expansion medium containing EGF or bFGF exhibited considerably increased von-Kossa staining, compared to the culture using the stromal medium. That is, according to the present invention, when the adipose-derived stromal stem cells are incubated in an expansion medium prepared by adding bFGF or EGF to the stromal medium, improved differentiation of the stem cells may be attained.

### EXAMPLE 4 - Immunomodulatory activity of human adipose-derived stromal stem cells

According to an analysis result of human leukocyte antigen (HLA), seven (7) types of human peripheral blood mononuclear cells (PBMC) having various combinations of Type I and II HLAs and adipose-derived stromal stem cells were selected, followed by implementation of mixture lymphocyte reaction (MLR). PBMCs contained in each lot, as responder cells, were added to each well of a U-bottom 96-well plate to be 2×10⁵ cells/well. Then, the adipose-derived stromal stem cells in each lot, as a stimulator, were added in an amount of 2×10⁴ cells to each well. As a positive control, 2×10⁴ PBMCs were treated using mitomycin C and added to the well. On day 3 after reaction, a supernatant was collected and subjected to measurement of an amount of secreted IFN-γ using ELISA method.

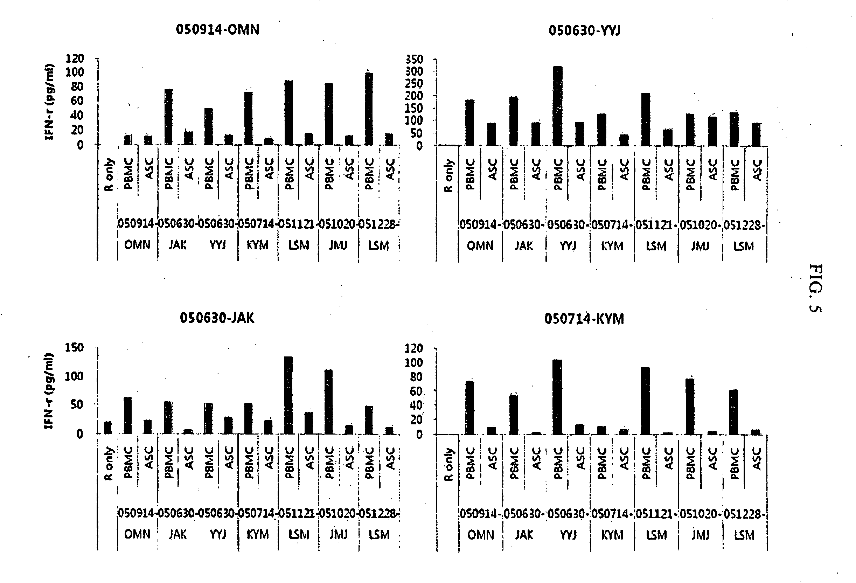

FIG. 5 illustrates immunogenicity of adipose stem cells. As shown in this figure, for PBMCs as the positive control, they reacted with allogenic PBMCs, resulting in secretion of a large amount of IFN-γ. On the other hand, the foregoing PBMCs reacted with the adipose-derived stromal stem cells cultured according to Example 1 did not exhibit IFN-γ secretion or exhibited IFN-γ in a level substantially similar to autologous PBMCs. That is, it can be seen that the adipose-derived stromal stem cells do not induce immune response to allogenic PBMCs.

According to another embodiment, two (2) lots containing different PBMCs were inoculated in an amount of 2×10⁵ cells/well into each well of a U-bottom 96-well plate to induce immune response, then, allogenic adipose-derived stromal stem cells cultured according to Example 1 were added in numbers of 5,000, 10,000 and 20,000, respectively, to the wells. After incubating for 72 hours, a supernatant was taken from each of the wells and subjected to measurement of an amount of IFN-γ secretion.

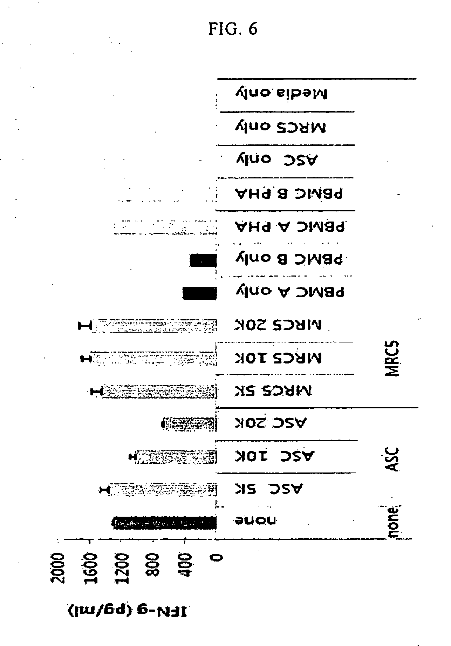

FIG. 6 illustrates immunosuppressive activity of adipose stem cells during MLR. As shown in this figure, it can be seen that the immune response is suppressed (or controlled) in proportion to the number of added cells when the adipose-derived stromal stem cells cultured according to Example 1 were added to the well. In other words, the adipose-derived stromal stem cells do not induce immune response by allogenic T cells and considerably decrease secretion of IFN-γ which is secreted in large quantities by immune cells when immune response occurs to thus increase immune activity, thereby having immunosuppressive activity.

### EXAMPLE 5 - Immunomodulatory activity of allogenic human adipose-derived stromal stem cells

In order to identify immunosuppressive activities of autologous and allogenic adipose-derived stromal stem cells, PBMCs obtained from four (4) different donors were activated using phyto-hemagglutinin (PHA) and subjected to measurement of amount of IFN-γ or TNF-α secretion. After the autologous or allogenic adipose-derived stromal stem cells were inoculated in an amount of 10,000 cells into each well of a 96-well plate, 1×10⁵ cells/well of PBMCs were added to the wells, followed by induction of immune response using PHA. After 72 hours, a supernatant was taken and subjected to measurement of the amount of IFN-γ or TNF-α secretion.

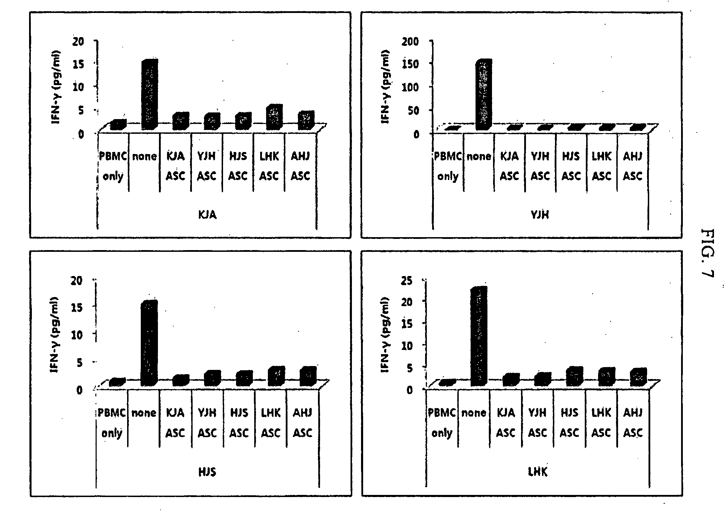

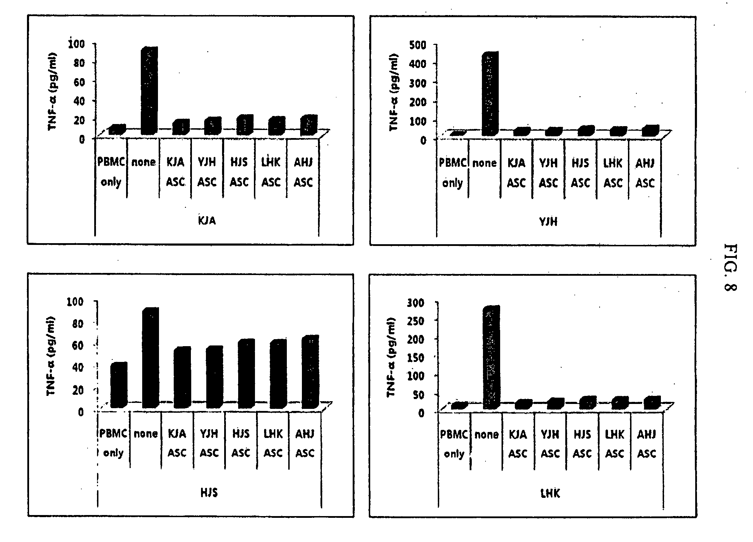

FIG. 7 illustrates IFN-γ secretion suppressive activity of autologous and allogenic adipose stem cells with respect to PHA-stimulated immune response, and FIG. 8 illustrates TNF-α secretion suppressive activity of autologous and allogenic adipose stem cells with respect to PHA-stimulated immune response. As shown in FIGS. 7 and 8, the amount of IFN-γ or TNF-α secretion was considerably decreased in both autologous and allogenic adipose-derived stromal stem cells when the adipose-derived stromal stem cells cultured according to Example 1 were added to the wells. Specifically, since the allogenic adipose-derived stromal stem cells exhibit substantially different immunomodulatory activities depending on types (individuals) thereof, excellent clinical effects may be attained in the case where a adipose-derived stromal cells having superior immunosuppressive activities are screened and used, compared to autologous stromal cells.

In other words, allogenic adipose-derived stem cells may control immune response induced by mitogen that activates immune cells, thus having immunosuppressive activity, and such activity is substantially equal to or better than that of autologous adipose-derived stem cells.

### EXAMPLE 6 - Immunomodulatory activity of human adipose-derived stromal stem cells produced by stromal medium or expansion medium

In order to compare immunomodulatory activity of human adipose-derived stromal stem cells cultured in a stromal medium or an expansion medium, adipose stem cells obtained from three (3) different donors were cultured using the stromal medium or the expansion medium disclosed in the present invention, respectively. PBMCs provided from three different donors were activated using PHA and then amounts of IFN-γ and TNF-α secretion were measured by adding the foregoing adipose stem cells to the activated PBMCs. After the adipose-derived stromal stem cells were inoculated in an amount of 10,000 cells into each well of a 96-well plate, 1×10⁵ cells/well of PBMCs were added to the wells, followed by induction of immune response using PHA. After 72 hours, a supernatant was taken and subjected to measurement of the amount of IFN-γ or TNF-α secretion.

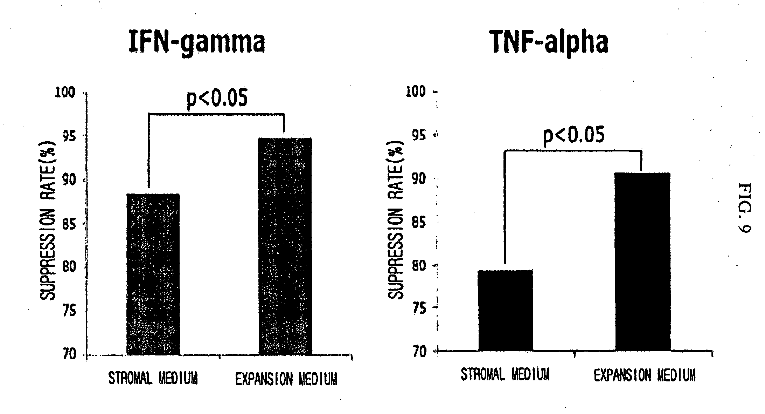

FIG. 9 illustrates both IFN-γ and TNF-α secretion suppressive activities (n=9) of adipose stem cells incubated in a stromal medium and an expansion medium, with respect to PHA-stimulated immune response. As shown in FIG. 9, it can be seen that, when the adipose-derived stromal stem cells cultured in the expansion medium according to Example 1 were added, IFN-γ and TNF-α secretion suppressive activities noticeably increased, compared to the stem cells produced using the stromal medium. That is, the adipose-derived stem cells produced according to the present invention more effectively suppressed (or controlled) immune response induced by mitogen, compared to existing standard culturing methods.

### EXAMPLE 7 - ECM protein secretion activity of human adipose-derived stromal stem cells

The adipose-derived stromal cells cultured in Example 1 were inoculated into each well of a four-well plate and placed in a culture vessel. The cells were washed three times with PBS and fixed using PBS containing 4% formaldehyde for 30 minutes. Then, after washing the fixed cells three times with PBS, the washed product was subjected permeabilization and blocking using PBS containing 5% normal goat serum and 0.1% Triton X-100 for 30 minutes. After PBS containing a primary antibody was added to the treated material and reacted at 37°C for 1 hour, the reaction product was washed three times using PBS and subjected to reaction using a secondary antibody for 30 minutes. After the product was washed again three times with PBS, the washed product was mounted by applying a mounting solution thereto and observed using a fluorescence microscope.

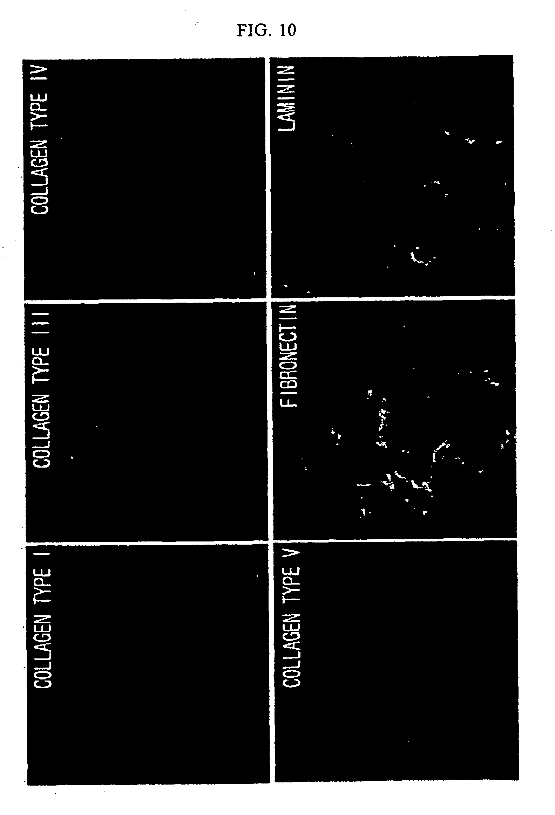

FIG. 10 is photographs (×40) showing extra-cellular matrix (ECM) protein secretion activity of adipose stem cells. As shown in the photographs, the adipose stem cells cultured according to the present invention exhibited positive response to collagen types I, III, IV and V, fibronectin and laminin. That is, the adipose-derived stromal cells cultured according to the present invention may express various types of ECMs such as collagen type I, III, IV and V, fibronectin, laminin, or the like, to thereby enable easier cell engraftment and/or formation of new tissue when the foregoing cells are transplanted into a body.

### EXAMPLE 8 - Illustrative example of treating Crohn's fistula using adipose-derived stromal cells

For clinical application of the adipose-derived stromal cells cultured by the same procedure as described in Example 1, clinical trials were conducted on patients with Crohn's fistula. The prepared cells were cultured for one to three passages. Then, the cells at a concentration of 1 to 2×10⁷ cells/ml were transplanted along a fistula wall in a volume of 4 to 14 ml depending on fistula size. After cell transplantation, the fisula was filled with fibrin glue.

### Case 1:

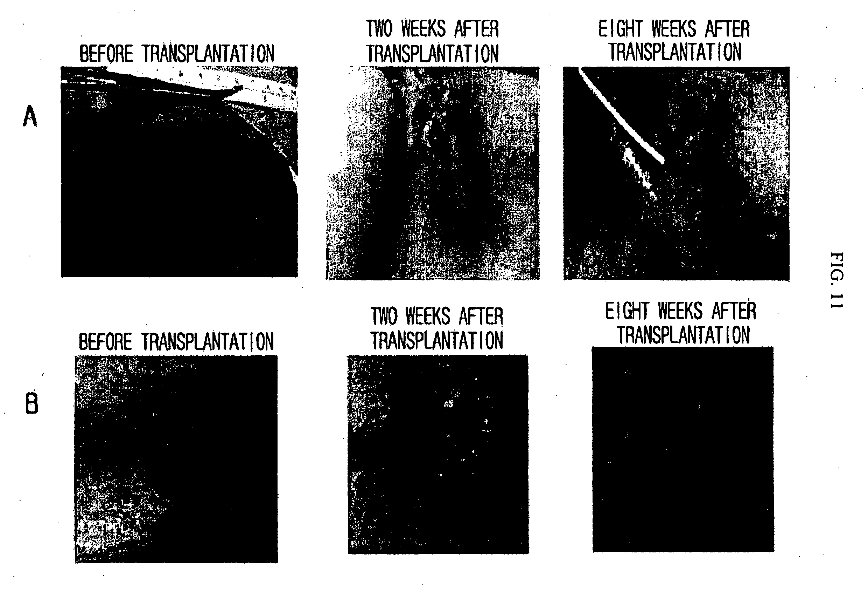

A 24 year-old female patient, was diagnosed with Crohn's disease 10 years ago and given a fistulectomy 2 years ago. However, one and a half years after the operation, the patient was subjected to surgical resection due to recurrence of the disease. In spite of administering Azathioprine, Remicade, etc., the state of disease did not improve. Due to continuous recurrence of fistula-in-ano and inflammation thereof, a size of the target fistula-in-ano was about 5.5cm(depth)×2cm(diameter), had an abscess discharging large amounts of pus, and got significantly worse (see A of FIG. 11). By taking about 50ml of adipose tissue from the abdomen of the patient, the adipose tissue was cultured for up to three passages, thereby obtaining a total of 2.8×10⁸ cells. An overall production period was 21 days. Before cell transplantation, a curettage was applied to remove inflamed tissue and cells at a concentration of 2×10⁷ cells/ml were transplanted along a fistula tract from the innermost side thereof. After transplantation, the fistula tract was filled with fibrin glue. From a follow-up observation for two weeks after cell transplantation, it was found that the fistula tract was almost closed and epithelization occurred on the majority of an external opening. As a result of eight (8) week follow-up observation, it was observed that the fistula-in-ano part was completely closed and the external opening was completely epithelized (complete closing of the inner fistula tract was identified by slightly scrapping the epithelized part).

### Case 2:

A 24 year-old male patient, was diagnosed with Crohn's disease 10 years ago and given a fistulectomy three times over the last 3 years. However, he had continuous recurrence of the disease and received repeated surgical resection. In spite of administering steroids, Remicade, etc., the state of disease did not improve. Due to continuous recurrence of fistula-in-ano and inflammation thereof, the target fistula-ion-ano was located at the 10 o'clock position at a distance of 1cm from the anus, had a size of 7 cm (depth)×1 cm (diameter) and abscess discharging large amounts of pus, and got significantly worse (see B of FIG. 11). By taking about 70ml of adipose tissue from the abdomen of the patient, the adipose tissue was cultured for up to three passages, thereby obtaining a total of 3×10⁸ cells. An overall incubation period was 20 days. Before cell transplantation, a curettage was applied to remove inflamed tissue and cells at a concentration of 2×10⁷ cells/ml were transplanted along a fistula tract from the innermost side thereof. After transplantation, the fistula tract was filled with fibrin glue. From a follow-up observation for two weeks after cell transplantation, it was found that at least half of the fistula tract, which had an original depth of about 7cm before cell transplantation, was closed, leaving a remaining depth of about 3cm. As a result of eight (8) weeks follow-up observation, it was observed that the fistula-in-ano part was completely closed and an external opening thereof was completely epithelized (complete closing of the inner fistula tract was identified by slightly scrapping the epithelized part).

FIG. 11 is photographs showing fistula-in-ano before and after transplantation of adipose stem cells.

As illustrated in the foregoing clinical examples, it can be seen that a fistula-in-ano, which does not respond to drugs and is continuously recurrent and difficult to treat using conventional methods, may be effectively treated using adipose stem cells cultured according to the inventive method. Especially, as described in Transplantation Cases 1 and 2, a method for treatment of a serious fistula-in-ano with, for example, a fistula tract having a large size such as a diameter of more than 1cm and/or a depth of more than 5cm has not yet been disclosed. For production of cells to be transplanted into a fistula-in-ano, large quantities of adipose stem cells could be cultured by employing an improved culturing method proposed in Example 1. The culturing method according to the present invention enabled provision of adipose tissue-derived stromal cells of more than 10 times than a conventional method disclosed in WO 2006/136244 (December 28, 2006), through culture for two or three passages. Furthermore, treatment results are noticeable, thereby attaining enhanced clinical effects.

### [Industrial Applicability]

A culturing method according to the present invention can effectively produce clinically effective number of adipose tissue-derived stromal stem cells within a short period by improving upon conventional standard culturing methods.

The adipose stem cell composition containing the adipose tissue-derived stromal stem cells obtained by the method of the present invention exhibits superior multipotency and immunomodulatory activity, as compared to cell compositions produced by conventional methods, and thus is more suitable for treating fistulas. Especially, allogenic adipose-derived stem cells are excellent in view of clinical applications.

## Claims

1. A method for production of an adipose stem cell composition comprising adipose-derived stromal stem cells, the method comprising the following steps:
(a) isolating a stromal vascular fraction (SVF) from adipose tissue collected from a subject;
(b) incubating the SVF in a stromal medium;
(c) incubating the SVF in an expansion medium containing EGF or bFGF as a growth factor, to culture the adipose-derived stromal stem cells; and
(d) culturing the adipose-derived stromal stem cells for at least one passage.

2. The method according to claim 1, wherein the expansion medium of process (c) comprises EGF or bFGF in a concentration of 0.1 to 100 ng/ml.

3. The method according to claim 2, wherein the expansion medium comprises EGF or bFGF in a concentration of 1 to 10 ng/ml.

4. The method according to claim 1, wherein the adipose tissue is collected from an allogenic subject.

## Patentansprüche

1. Verfahren zur Herstellung einer Fettgewebe-Stammzellenzusammensetzung, aufweisend Fettgewebe-abgeleitete, stromale Stammzellen, wobei das Verfahren die folgenden Schritte aufweist:
(a) Isolierung einer stromalen vaskulären Fraktion (SVF) aus Fettgewebe, das bei einem Probanden gesammelt wurde,
(b) Inkubieren der SVF in einem stromalen Medium,
(c) Inkubieren der SVF in einem Expansionsmedium, das EGF oder bFGF als einen Wachstumsfaktor enthält, um die von Fettgewebeabgeleiteten, stromalen Stammzellen zu kultivieren, und
(d) Kultivieren der von Fettgewebe-abgeleiteten, stromalen Stammzellen für mindestens eine Passage.

2. Verfahren nach Anspruch 1, wobei das Expansionsmedium von Prozess (c) EGF oder bFGF in einer Konzentration von 0,1 bis 100 ng/ml aufweist.

3. Verfahren nach Anspruch 2, wobei das Expansionsmedium EGF oder bFGF in einer Konzentration von 1 bis 10 ng/ml aufweist.

4. Verfahren nach Anspruch 1, wobei das Fettgewebe bei einem allogenen Probanden gesammelt wird.

## Revendications

1. Procédé de production d'une composition de cellules souches adipeuses comprenant des cellules souches stromatolithiques dérivées d'adipose, le procédé comprenant les étapes suivantes :
(a) isoler une fraction vasculaire stromatolithique (SVF) à partir de tissu adipeux recueilli auprès d'un sujet ;
(b) incuber la SVF dans un milieu stromatolithique ;
(c) incuber la SVF dans un milieu d'expansion contenant EGF ou bFGF comme facteur de croissance pour la mise en culture des cellules souches stromatolithiques dérivées d'adipose ; et
(d) mettre en culture les cellules souches stromatolithiques dérivées d'adipose pendant au moins un passage.

2. Procédé selon la revendication 1, dans lequel le milieu d'expansion du processus (c) comprend EGF ou bFGF à une concentration de 0,1 à 100 ng/ml.

3. Procédé selon la revendication 2, dans lequel le milieu d'expansion comprend EGF ou bFGF à une concentration de 1 à 10 ng/ml.

4. Procédé selon la revendication 1, dans lequel le tissu adipeux est recueilli auprès d'un sujet allogénique.
